# EUROPEAN PATENT SPECIFICATION

(11) **EP 2 313 034 B1**
(45) Date of publication and mention of the grant of the patent: **23.09.2015**
(21) Application number: 09774546.7
(22) Date of filing: 02.07.2009
(51) Int. Cl.: A61F 5/00, A61M 31/00, A61M 37/00, A61F 2/80

(54) **Devices for delivering or delaying lipids within a duodenum**
Vorrichtung für die Abgabe oder Verzögerung von Lipiden innerhalb eines Duodenums
Dispositifs d'introduction ou de ralentissement des lipides dans le duodénum

(30) Priority: 02.07.2008 US 77579 P; 16.07.2008 WO PCT/US2008/070226
(43) Date of publication of application: 27.04.2011
(73) Proprietor: Endosphere, Inc., Columbus OH 43219 (US)
(72) Inventor: McKINLEY, James T., Redwood City CA 94065 (US); KECK, Zhenyong, Redwood City CA 94065 (US); SANDER, Fiona M., Redwood Cit CA 94065 (US)
(74) Representative: Potter Clarkson LLP
(86) International application number: PCT/US2009/049586
(87) International publication number: WO 2010/003097

(56) References cited:
- WO-A1-2009/012335
- US-A- 4 315 509
- US-A- 5 396 880
- US-A1- 2002 002 384
- US-A1- 2005 049 718
- US-A1- 2005 192 614
- US-A1- 2007 135 768
- US-A1- 2007 156 159
- US-A1- 2007 293 885
- US-B1- 6 306 163

## Description

### BACKGROUND OF THE INVENTION

Obesity and type 2 diabetes are diseases of insufficient or deficient regulation. We know from human studies that the small intestine plays a critical role in both energy and glucose homeostasis: when the duodenum is exposed to lipids, appetite is diminished and native (liver) glucose production is down-regulated. Previously we have invented a device that can be implanted into the duodenum and remain in place; we have described devices and methods for slowing the passage of food through the duodenum to cause increased tissue-nutrient contact, thereby causing amplified hormonal signaling from the duodenum; and, we have invented methods and devices for delivering chemicals, drugs or other compounds to the duodenum.

US 2007/0293885 discloses a device for curbing appetite and/or reducing food intake according to the preamble of claim 1. The device comprises an intestinal insert having an elongated member and at least one flow reduction element positioned along the elongated member.

US 2005/0049718 discloses a device for treating morbid obesity. The device comprises a gastric sleeve device, an intestinal sleeve device or a combined gastrointestinal sleeve device.

US 2007/0156159 discloses a device for dividing a hollow body organ or otherwise restricting or partitioning a certain section of that organ, such as a stomach, intestine or gastrointestinal tract. The device comprises a main body having at least one lumen therethrough.

US-4,315,509 discloses a device for lining a portion of the human alimentary canal comprising a balloon-like tube.

US 2005/0192614 discloses a device for treating morbid obesity comprising a series of flow reduction elements positioned on an elongated tube.

WO 2009/012335 discloses a device for curbing appetite or reducing food intake comprising a central spine, a plurality of flow reduction elements positioned on the central spine and atraumatic features at proximal and distal ends of the spine.

What is needed are devices for additional lipid uptake, delaying ingesta passage/prolonging ingesta contact, delivering or providing lipids or other appetite and glucose reducing nutrients and/or combinations of these characteristics.

### SUMMARY OF THE INVENHON

The present invention provides a device configured for use within the duodenum of a human according to claim 1. The device has a spine having a proximal end and a distal end; an atraumatic feature positioned on at least one of the proximal end and the distal end of the spine; and, positioned along the spine, a flow reduction element having variable porosity along its length. The variable porosity of the flow reduction element is selected so that a portion of a flow over the proximal end of the flow reduction element flows into an interior portion of the flow reduction element. In one embodiment, the variable porosity of the flow reduction element is selected so that flow within an interior of the flow reduction element is at least partially inhibited from flowing through the distal portion of the variable porosity structure. In one embodiment, the proximal portion of the flow reduction element comprises a material, a mesh or a braid having a porosity or altered to provide a porosity selected to permit a flow into an interior portion of the flow reduction element. In one embodiment, the distal portion of the flow reduction element comprises a material, a mesh or a braid having a porosity or altered to provide a porosity selected to at least partially inhibit a flow from within an interior portion of the flow reduction element. In one embodiment, the length of the spine is selected so when the atraumatic feature is positioned in a stomach the flow reduction element is positioned on the spine and distal to a pylorus. In one embodiment, the length of the spine is selected so when the atraumatic feature is positioned in a stomach the distal end of the spine is in the fourth portion of the duodenum and the flow reduction element is positioned within a portion of the duodenum. In one embodiment, there is also a flow reduction element having a non-variable porosity along its length. In one embodiment, there is also a feature on the spine positioned to restrict movement of the flow reduction element relative to the spine.

In one embodiment, the device has a first atraumatic feature positioned adjacent to the spine proximal end and a second atraumatic feature positioned adjacent to the spine distal end. In one particular embodiment, the length of the spine is selected so that when the first atraumatic feature is in the stomach the second atraumatic feature is in the fourth portion of the duodenum. In one embodiment, the distal end of the flow reduction element has a solid portion to retain material within the interior portion. In one embodiment, there is provided another flow reduction element between the proximal and distal ends of the spine. In one embodiment, the another flow reduction element has variable porosity between the proximal and distal ends of the flow reduction element. In one particular embodiment, the length of the spine is selected so that when the first atraumatic feature is positioned in a stomach the second atraumatic feature is positioned within the duodenum and adjacent to the first atraumatic feature. In one particular embodiment, the length of the spine is selected so that when the first atraumatic feature is positioned in a stomach the second atraumatic feature is positioned within the duodenum and in proximity to the ligament of Treitz.

Also described but not claimed is a method for adjusting the passage of ingesta through the duodenum of a mammal by placing the distal end of a flow reduction device in the duodenum of a mammal; placing the proximal end of the flow reduction device in a stomach of the mammal; expanding within the duodenum of the mammal a variable porosity flow reduction element supported by the flow reduction device; and directing at least a portion of the flow of ingesta through the duodenum through the proximal end of the variable porosity flow reduction element and into an interior portion of the variable porosity flow reduction element. In one method, also not claimed, a portion of the ingesta is retained within the interior portion of the variable porosity flow reduction element until the ingesta is pushed out of the interior portion by peristaltic action of the duodenum. Also described but not claimed is a method of adjusting the passage of ingesta wherein the ingesta is pushed out of the interior portion by passing through a distal portion of the variable porosity flow reduction element. In still another alternative method for adjusting the passage of ingesta, also not claimed, the ingesta is pushed out of the interior portion by passing through the proximal portion and thereafter passing around the variable porosity flow reduction element.

In another embodiment, the flow reduction elements positioned along the device backbone could be coated with, manufactured from or contain lipid-philic materials. For example, one or more of the flow reduction elements of the intraduodenal device could be manufactured from a lipid absorbing or adsorbing material. A flow reduction element manufactured in this way will attract and collect lipids from passing ingesta and hold the lipids for a period of time. The length of time that the lipids will remain attached to the flow reduction element will vary based on a number of factors. For example, the flow reduction element may exude the lipids as peristalsis continues to squeeze the device. The prolonged presence of and/or sustained release of lipids within the duodenum is believed to benefit and in some cases to increase hormonal regulation.

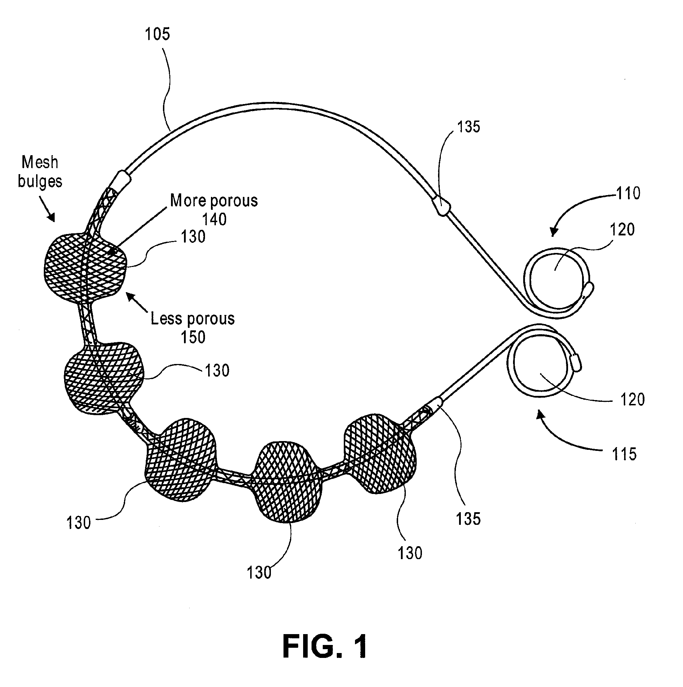

It is to be appreciated that embodiments of the present invention provide prolonged presence of and/or sustained release of lipids within the duodenum through the use of one or more of: making flow reduction elements and/or other components of the flow reduction device (i.e., any of the components of FIG. 1 for example) at least partially or even completely from one or more lipid-philic materials; coating flow reduction elements and/or other components of the flow reduction device entirely or at least partially form lipid-philic materials; and/or placing within a flow reduction element, or attaching to a component of the flow reduction device an additional element or component formed from or at least partially coated with a lipid-philic material.

### BRIEF DESCRIPTION OF THE DRAWINGS

FIG 1 is a prospective view of a device for use in the duodenum.
FIG. 2 is an enlarged view of one of the flow reduction elements of FIG. 1 showing the upper and lower portions of the element;
FIG. 2A is an enlarged view of a material used to provide variable porosity to a flow portion of a flow reduction element;
FIG. 2B is an enlarged view of a solid or semi permeable sheet where a pattern of holes are used to provided variable porosity to a portion of a flow reduction element;
FIG. 3 illustrates a flow reduction element embodiment where more of the flow reduction device is utilized for allowing flow through the use of a larger proximal portion having a more porous construction and a smaller distal portion having a less porous construction;
FIG. 4 illustrates a flow reduction element embodiment where more of the flow reduction device is utilized for preventing flow than for allowing flow through the use of a smaller proximal portion having a more porous construction and a larger distal portion having a less porous construction.

### DETAILED DESCRIPTION OF THE INVENTION

The present application relates to devices for providing a sense of fullness/satiety and/or glucose production inhibition. The devices and exemplary methods described herein provide for additional lipid presence in the duodenum by delaying ingesta passage/prolonging ingesta contact, delivering or providing lipids or other satiety and/or glucose reducing nutrients and/or combinations thereof.

Lipids can be generally defined as substances such as a fat, oil or wax that dissolve in alcohol but not in water. Lipids contain carbon, hydrogen and oxygen but have far less oxygen proportionally than carbohydrates. They serve as a source of fuel and are an important constituent of the structure of cells. As used herein, lipids refer to any of fatty acids, glycerides, complex lipids and nonglycerides. Fatty acids include both saturated and unsaturated. Glycerides include, for example, neutral phosphoglycerides. Complex lipids are lipids complexed with another type of chemical compound and include, for example, lipoproteins, phospholipids and glycolipids. Nonglycerides include, for example, sphingolipds, steroids and waxes.

Because most ingesta contain lipids, it is believed that the methods and devices described herein for prolonging lipid residence time, delaying lipid passage and/or providing lipids would enhance the operating characteristics of a duodenal positioned flow reduction device. In other words, the methods and devices described herein not only slow the passage of food but may also detain or delay the passage of food. The delay or detention may allow for prolonged nutrient release and aid in achieving health objectives. Additionally or alternatively, the devices and methods described herein may also provide for active introduction of lipids into the duodenum.

By using flow reduction bulges along the spine of the device pictured in Fig. 1, ingesta can be caught and held temporarily. The mesh bulges may have a more open structure on the end from which nutrients arrive from the stomach and smaller openings on the end from which nutrients depart as one way to hold nutrients longer. Alternatively, the spheres could themselves become 'buckets' in which food is captured and retained. The bucket may be provided by having a solid bottom portion so that chyme entering remains in the bottom of the bucket until pushed out by peristaltic action or my movement of additional chyme to displace it.

The flow restriction devices may be any of a wide variety of shapes and dimensions. The adjustment of the flow characteristics of ingesta or chyme: (a) around the outside of a flow reduction element, (b) through a wall of a flow or portion of a flow reduction element and (c) retained within a flow reduction element may be accomplished in a number of ways. One way to adjust the flow characteristics within a flow regulated duodenum (i.e., a duodenum having a flow reduction device implanted within it) is through the porosity or flow characteristics of the flow reduction elements.

FIG. 1 illustrates a perspective view of a device 100 having a spine 105, a proximal end 110, a distal end 115, atraumatic features 120 on both the proximal and distal ends. A series of five flow reduction elements 130 are shown in position along the spine 105. There maybe one or more features 135 on the spine to prevent unwanted proximal or distal movement of the flow reduction elements 130 along the spine 105. The flow reduction elements 130 are shown in a deployed configuration as they would be.in use within the duodenum. In the most basic form, one or more of the flow reduction elements may be formed from a material or materials with porosity characteristics that adjust the flow characteristics of chyme or ingesta flow relative to the material and the flow reduction element.

As shown in FIG. 1, the device 100 is orientated as it would be implanted with the proximal end 110 in the stomach and the distal end 115 in the duodenum near the ligament of Trietz. Chyme/ingesta flow is from the proximal end 110 to the distal end 115 in FIG. 1. The more porous portion 140 of the flow reduction element 130 will interact with the chyme first. Because of the porosity of this part of the flow reduction element, chyme will flow into the interior portion of the flow reduction element as well as around the flow reduction element. As peristaltic action in the duodenum continues to move the chyme, the portion inside of the flow reduction element is urged distally towards the portion of the flow reduction device that is less porous 150. Some of the chyme entering the flow reduction element portion 150 will be retained within the flow reduction element for some period of time based upon a number of factors such as how much the person has eaten and the porosity of this portion of the flow reduction element among others. As a result of the variable porosity of the structure of the flow reduction element, chyme enters but then is delayed in leaving the flow reduction element interior. It is believed that increasing the residence time of chyme within a flow reduction element will increase the time of interaction of the nutrients in that trapped portion and/or provide for an extended release of the lipids in the trapped portion of chyme. The retained food could be nutrient rich or lipid rich so that the prolonged exposure produces a sense of satiety and/or the inhibition of liver glucose production in the individual. One such mechanism that may be achieved or fostered by the embodiments described herein is described in "Upper intestinal lipids trigger a gut-brain-liver axis to regulate glucose production" by Penny Y.T. Wang et al (Nature, Vol. 452:24 April 2008 doi:10.1038/nature06852).

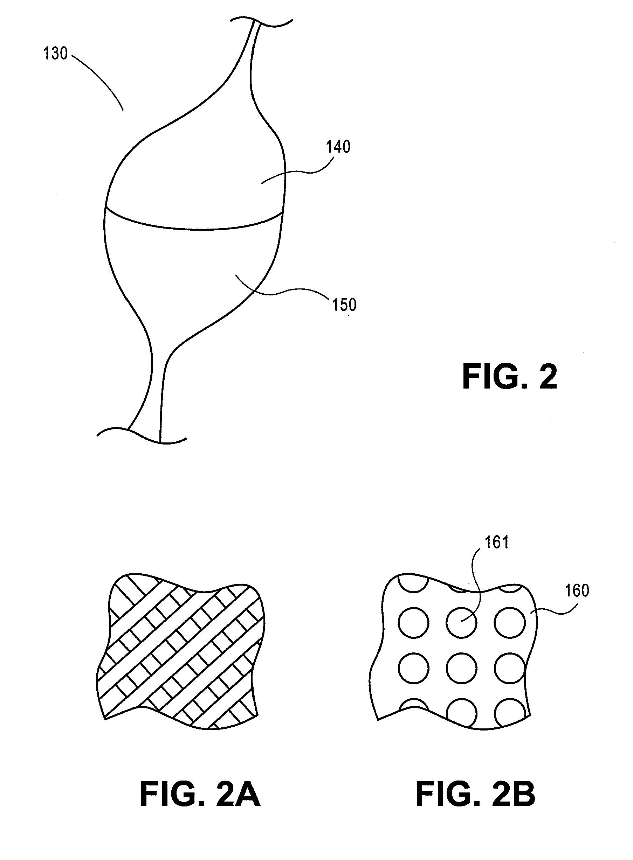

The flow characteristics of the flow reduction element may be adjusted in a number of ways. FIG. 2 is an enlarged view of a flow reduction element 130 in FIG. 1. The flow reduction element is divided roughly in half with an upper portion 140 (the part to first contact with the flow of chyme) having a porosity that allows flow through it and into the interior of the flow reduction element. The bottom portion 150 of the flow reduction element is less porous or allows less flow of chyme from the interior of the flow reduction element. The variable porosity or flow characteristics of the flow reduction elements may be adjusted by selecting material with different sizes (FIG. 2A), shapes, construction, and/or filaments with different characteristics to enhance or diminish flow as needed for the upper or lower portions. The flow characteristics of a flow reduction element may be obtained by overlapping (i.e., joined or crossed without joining) filaments to form cells that will, to the desired degree, permit or impede chyme passage. Alternatively or additionally, the flow reduction element could be formed from a solid or semipermeable sheet 160 with holes 161 formed in it as shown in FIG. 2B. The size, shape, pattern and distribution of the openings may be used to adjust the amount of relative flow through a flow reduction element.

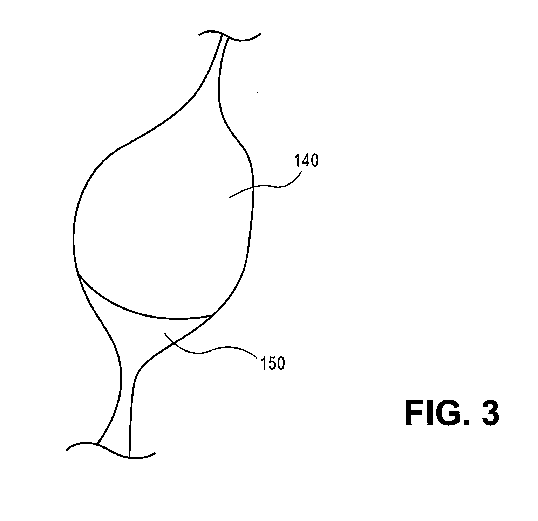

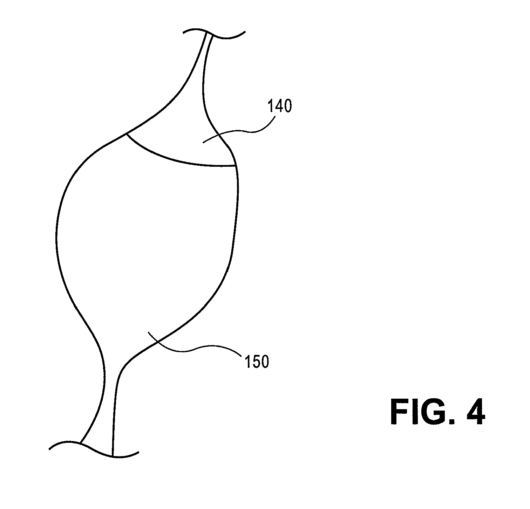

The flow reduction element adjustment may also be accomplished by adjusting the relative amounts of the flow reduction element used for allowing flow (more porous 140) or preventing flow (less porous 150). FIG. 3 illustrates a flow reduction element embodiment where more of the flow reduction device is utilized for allowing flow than for preventing flow. In contrast, FIG. 4 illustrates a flow reduction element embodiment where more of the flow reduction device is utilized for preventing flow than for allowing flow.

The foregoing are merely examples. More than one flow allowing or more than one flow preventing zone or portion may be used in a flow reduction element. While the relative distribution of the flow zones has been generally perpendicular to the spine or flow within the duodenum, other orientations relative to the spine or the duodenum are possible depending upon the desired flow profile. Moreover, a device may have flow reduction elements having one or more flow adjustment zones, or no flow adjustment zones. The type of flow reduction elements and the flow characteristics of those flow reduction elements may be mixed within a flow reduction device depending upon the desired flow characteristics desired.

Other flow reduction element properties such as the shape and size of a reduction element, the relative size between reduction elements, the alignment or orientation of a reduction element to the central shaft or spine and other properties
may also be adjusted to accomplish the nutrient delivery aspects described herein.

The flow reduction elements could also be configured to capture and hold a lipid 'pill'. For instance, to maintain a certain lipid level in the spheres, the patient could periodically swallow a lipid 'pill' or other form that engages the device and remains in place. The nutrient could also take the form of an extended release compound that releases an amount of the nutrient over time. The nutrient could also be mixed with another compound that is released when the patient consumes a releasing agent, such as before a meal or at a time of day prone to hunger pangs or higher than desired blood glucose levels. The releasing agent mixes with the nutrient compound mixture causing the release of nutrients, such as lipids, to aid in regulation of hunger and/or glucose.

In another aspect, the flow reduction device could be coated with, manufactured with or contain nutrients such as lipids or lipid-philic materials. For example, the flow reduction elements could be one or more bulges manufactured using lipid leaching materials; or the bulges could be manufactured with lipid-philic materials or a sponge like membrane inside the bulges or otherwise connected to the device backbone, that would absorb and distribute temporally lipids from passing ingesta to increase hormonal regulation.

In another aspect, the flow reduction elements positioned along the device backbone could be coated with, manufactured from or contain lipid-philic materials. For example, one or more of the flow reduction elements of the intraduodenal device could be manufactured from a lipid absorbing or adsorbing material. A flow reduction element manufactured in this way will attract and collect lipids from passing ingesta and hold the lipids for a period of time. The length of time that the lipids will remain attached to the flow reduction element will vary based on a number of factors. For example, the flow reduction element may exude the lipids as peristalsis continues to squeeze the device. The prolonged presence of and/or sustained release of lipids within the duodenum is believed to benefit and in some cases to increase hormonal regulation. It is to be appreciated that embodiments of the present invention provide prolonged presence of and/or sustained release of lipids within the duodenum through the use of one or more of: making flow reduction elements and/or other components of the flow reduction device (i.e., any of the components of FIG. 1 for example) at least partially or even completely from one or more lipid-philic materials; coating flow reduction elements and/or other components of the flow reduction device entirely or at least partially form lipid-philic materials; and/or placing within a flow reduction element, or attaching to a component of the flow reduction device an additional element or component formed from or at least partially coated with a lipid-philic material.,

The spine could be configured as a central tube with plural inlet/outlet ports in communication with a flow reduction element and/or the outside of the tube. Additionally or alternatively, a portion of the device itself, i.e.: the backbone or spine, could be hollow such that it could be filled with lipids that would leach out or otherwise be delivered to the duodenum. A nutrient reservoir may be implanted within the patient, within the flow reduction device or separately injected into the device during implantation, after insertion or periodically while the patient has the device inserted in his duodenum.

Additionally or alternatively, the lipid equipped hormonal regulatory device could be configured to release lipids on demand using an internal or external controller or device as described in U.S. Patent Application No. 11/807,107, filed May 25,2007, entitled "METHODS AND DEVICES TO CURB APPETITE AND/OR REDUCE FOOD INTAKE," now publication no. 2007-0293885.

## Claims

1. A device (100) configured for use within the duodenum of a human, comprising:
a spine (105) having a proximal end (110) and a distal end (115);
an atraumatic feature (120) positioned on at least one of the proximal end and the distal end of the spine; and
a porous flow reduction element (130) positioned along the spine having a surrounding wall defining a hollow interior,
**characterized in that** the wall of the flow reduction element has a variable porosity along its length such that the wall at a proximal end (140) of the flow reduction element is more porous than the wall at a distal end (150) of the flow reduction element and **in that** the variable porosity of the wall of the flow reduction element (130) is such that a portion of a flow over the proximal end (140) of the flow reduction element can flow into an interior portion of the flow reduction element.

2. The device of claim 1, wherein the variable porosity of the wall of the flow reduction element (130) is selected so that flow within the interior of the flow reduction element is at least partially inhibited from flowing through the distal portion (150) of the variable porosity structure.

3. The device of claim 1, wherein the wall at the proximal end (140) of the flow reduction element (130) comprises a material, a mesh or a braid having a porosity or altered to provide a porosity selected to permit a flow into an interior portion of the flow reduction element.

4. The device of claim 1, wherein the wall at the distal end (150) of the flow reduction element (130) comprises a material, a mesh or a braid having a porosity or altered to provide a porosity selected to at least partially inhibit a flow from within an interior portion of the flow reduction element.

5. The device of claim 1, wherein the length of the spine (105) is selected so when the atraumatic feature (120) is positioned in a stomach the flow reduction element (130) is positioned distal to a pylorus.

6. The device of claim 1, wherein the length of the spine (105) is selected so when the atraumatic feature (120) is positioned in a stomach the distal end (115) of the spine is in the fourth portion of the duodenum and the flow reduction element (130) is positioned within a portion of the duodenum.

7. The device of claim 1 further comprising:
a flow reduction element (130) having a non-variable porosity along its length.

8. The device of claim 7, wherein the or each flow reduction element (130) is at least partially formed from a lipid-philic material.

9. The device of claim 7, wherein at least a portion of the interior or an exterior of the or each flow reduction element (130) is at least partially coated with a lipid-philic material.

10. The device of claim 1 further comprising:
a feature (135) on the spine (105) positioned to restrict movement of the flow reduction element (130) relative to the spine.

11. The device of claim 1, wherein the flow reduction element (130) is at least partially formed from a lipid-philic material.

12. The device of claim 1, wherein at least a portion of the interior or an exterior of the flow reduction element (130) is at least partially coated with a lipid-philic material.

13. The device of claim 1 further comprising:
a lipid-philic structure within the flow reduction element (130).

## Patentansprüche

1. Vorrichtung (100), die zur Verwendung im Zwölffingerdarm eines Menschen gestaltet ist, umfassend:
ein Rückgrat (105) mit einem proximalen Ende (110) und einem distalen Ende (115),
ein atraumatisches Merkmal (120), das am proximalen Ende und/oder am distalen Ende des Rückgrats angeordnet ist, und
ein poröses Durchflussreduktionselement (130), das entlang des Rückgrats angeordnet ist und eine umgebende Wand aufweist, die einen hohlen Innenraum definiert,
**dadurch gekennzeichnet, dass** die Wand des Durchflussreduktionselements über ihre Länge eine variable Porosität aufweist, so dass die Wand an einem proximalen Ende (140) des Durchflussreduktionselements poröser ist als die Wand an einem distalen Ende (150) des Durchflussreduktionselements, und dadurch, dass die variable Porosität der Wand des Durchflussreduktionselements (130) derart ist, dass ein Anteil eines Durchflusses über das proximale Ende (140) des Durchflussreduktionselements in einen Innenabschnitt des Durchflussreduktionselements fließen kann.

2. Vorrichtung nach Anspruch 1, wobei die variable Porosität der Wand des Durchflussreduktionselements (130) derart ausgewählt ist, dass ein Durchfluss im Inneren des Durchflussreduktionselements zumindest teilweise daran gehindert wird, durch den distalen Abschnitt (150) der Struktur mit variabler Porosität zu fließen.

3. Vorrichtung nach Anspruch 1, wobei die Wand am proximalen Ende (140) des Durchflussreduktionselements (130) ein Material, ein Netz oder ein Geflecht umfasst, das eine Porosität aufweist oder derart geändert ist, dass es eine Porosität bereitstellt, die so gewählt ist, dass ein Durchfluss in einen Innenabschnitt des Durchflussreduktionselements ermöglicht wird.

4. Vorrichtung nach Anspruch 1, wobei die Wand am distalen Ende (150) des Durchflussreduktionselements (130) ein Material, ein Netz oder ein Geflecht umfasst, das eine Porosität aufweist oder derart geändert ist, dass es eine Porosität bereitstellt, die so gewählt ist, dass ein Durchfluss von innerhalb eines Innenabschnitts des Durchflussreduktionselements zumindest teilweise gehemmt wird.

5. Vorrichtung nach Anspruch 1, wobei die Länge des Rückgrats (105) so gewählt ist, dass, wenn das atraumatische Merkmal (120) in einem Magen angeordnet ist, das Durchflussreduktionselement (130) distal zu einem Magenpförtner angeordnet ist.

6. Vorrichtung nach Anspruch 1, wobei die Länge des Rückgrats (105) so gewählt ist, dass, wenn das atraumatische Merkmal (120) in einem Magen angeordnet ist, sich das distale Ende (115) des Rückgrats im vierten Abschnitt des Zwölffingerdarms befindet und das Durchflussreduktionselement (130) innerhalb eines Abschnitts des Zwölffingerdarms angeordnet ist.

7. Vorrichtung nach Anspruch 1, ferner umfassend:
ein Durchflussreduktionselement (130) mit einer nicht-variablen Porosität über seine Länge.

8. Vorrichtung nach Anspruch 7, wobei das oder jedes Durchflussreduktionselement (130) zumindest teilweise aus einem lipidophilen Material gebildet ist.

9. Vorrichtung nach Anspruch 7, wobei zumindest ein Abschnitt des Inneren oder Äußeren des oder jedes Durchflussreduktionselements (130) zumindest teilweise mit einem lipidophilen Material beschichtet ist.

10. Vorrichtung nach Anspruch 1, ferner umfassend:
ein Merkmal (135) an dem Rückgrat (105), das so angeordnet ist, dass es die Bewegung des Durchflussreduktionselements (130) in Bezug auf das Rückgrat einschränkt.

11. Vorrichtung nach Anspruch 1, wobei das Durchflussreduktionselement (130) zumindest teilweise aus einem lipidophilen Material gebildet ist.

12. Vorrichtung nach Anspruch 1, wobei zumindest ein Abschnitt des Inneren oder Äußeren des Durchflussreduktionselements (130) zumindest teilweise mit einem lipidophilen Material beschichtet ist.

13. Vorrichtung nach Anspruch 1, ferner umfassend:
eine lipidophile Struktur innerhalb des Durchflussreduktionselements (130).

## Revendications

1. Dispositif (100) configuré pour être utilisé à l'intérieur du duodénum d'un humain, comprenant :
une échine (105) ayant une extrémité proximale (110) et une extrémité distale (115) ;
une particularité atraumatique (120) positionnée sur au moins l'une de l'extrémité proximale et de l'extrémité distale de l'échine; et
un élément de réduction d'écoulement poreux (130) positionné le long de l'échine ayant une paroi périphérique définissant un intérieur creux,
**caractérisé en ce que** la paroi de l'élément de réduction d'écoulement a une porosité variable sur sa longueur de sorte que la paroi au niveau d'une extrémité proximale (140) de l'élément de réduction d'écoulement est plus poreuse que la paroi au niveau d'une extrémité distale (150) de l'élément de réduction d'écoulement et **en ce que** la porosité variable de la paroi de l'élément de réduction d'écoulement (130) est telle qu'une partie d'un écoulement sur l'extrémité proximale (140) de l'élément de réduction d'écoulement puisse se faire dans une partie intérieure de l'élément de réduction d'écoulement.

2. Dispositif selon la revendication 1, dans lequel la porosité variable de la paroi de l'élément de réduction d'écoulement (130) est choisie de sorte que l'écoulement à l'intérieur de l'élément de réduction d'écoulement soit au moins partiellement inhibé à travers la partie distale (150) de la structure à porosité variable.

3. Dispositif selon la revendication 1, dans lequel la paroi au niveau de l'extrémité proximale (140) de l'élément de réduction d'écoulement (130) comprend un matériau, une maille ou une tresse ayant une porosité ou étant modifié pour fournir une porosité choisie pour permettre un écoulement dans une partie intérieure de l'élément de réduction d'écoulement.

4. Dispositif selon la revendication 1, dans lequel la paroi au niveau de l'extrémité distale (150) de l'élément de réduction d'écoulement (130) comprend un matériau, une maille ou une tresse ayant une porosité ou étant modifié pour fournir une porosité choisie afin d'inhiber au moins partiellement un écoulement depuis l'intérieur d'une partie intérieure de l'élément de réduction d'écoulement.

5. Dispositif selon la revendication 1, dans lequel la longueur de l'échine (105) est choisie de sorte que lorsque la particularité atraumatique (120) est positionnée dans un estomac, l'élément de réduction d'écoulement (130) soit positionné de façon distale à un pylore.

6. Dispositif selon la revendication 1, dans lequel la longueur de l'échine (105) est choisie de sorte que lorsque la particularité atraumatique (120) est positionnée dans un estomac, l'extrémité distale (115) de l'échine soit dans la quatrième partie du duodénum et que l'élément de réduction d'écoulement (130) soit positionné à l'intérieur d'une partie du duodénum.

7. Dispositif selon la revendication 1, comprenant en outre :
un élément de réduction d'écoulement (130) ayant une porosité non variable sur sa longueur.

8. Dispositif selon la revendication 7, dans lequel le ou chaque élément de réduction d'écoulement (130) est au moins partiellement formé d'un matériau lipophile.

9. Dispositif selon la revendication 7, dans lequel au moins une partie de l'intérieur ou d'un extérieur du ou de chaque élément de réduction d'écoulement (130) est au moins partiellement revêtue d'un matériau lipophile.

10. Dispositif selon la revendication 1, comprenant en outre :
une particularité (135) sur l'échine (105) positionnée pour restreindre le mouvement de l'élément de réduction d'écoulement (130) par rapport à l'échine.

11. Dispositif selon la revendication 1, dans lequel l'élément de réduction d'écoulement (130) est au moins partiellement formé d'un matériau lipophile.

12. Dispositif selon la revendication 1, dans lequel au moins une partie de l'intérieur ou d'un extérieur de l'élément de réduction d'écoulement (130) est au moins partiellement revêtue d'un matériau lipophile.

13. Dispositif selon la revendication 1, comprenant en outre :
une structure lipophile à l'intérieur de l'élément de réduction d'écoulement (130).
